# EUROPEAN PATENT APPLICATION

(11) **EP 3 378 515 A1**
(43) Date of publication of application: **26.09.2018**
(21) Application number: 16866559.4
(22) Date of filing: 13.10.2016
(51) Int. Cl.: A61M 5/32, A61M 5/34, A61M 5/50

(54) **SYRINGE SAFETY CAP ASSEMBLY**

(30) Priority: 16.11.2015 KR 20150160136; 25.01.2016 KR 20160008504
(71) Applicant: Uniteko Co., Ltd., Gyeonggi-do 12732 (KR)
(72) Inventor: LEE, Hyunseung, Guri-si Gyeonggi-do 11930 (KR)
(74) Representative: Zardi, Marco
(86) International application number: PCT/KR2016/011513
(87) International publication number: WO 2017/086607

(57) **Abstract**

Provided is a syringe safe cap assembly and, more particularly, a syringe safe cap assembly for preventing user injury when a syringe needle is inserted into a cap and for originally preventing reuse of a syringe or a syringe needle that needs to be discarded. The syringe safe cap assembly includes an accommodation cap into which at least a portion of a syringe needle or a syringe needle support is capable of being inserted and accommodated, and a support connected to the accommodation cap and configured to support the accommodation cap not to fall down with respect to a ground surface, wherein the supports includes a housing portion, at least a portion of an external surface of which has an outline of a curved line, and when the accommodation cap is put on a ground, the support guides the accommodation cap to immediately stand on a ground or, selectively, guides the accommodation cap to lastly stand on the ground while the accommodation cap rolls from side to side.

## Description

### [Technical Field]

The present invention relates to a syringe safe cap assembly, and more particularly, to a syringe safe cap assembly for preventing user injury when a syringe needle is inserted into a cap and for originally preventing reuse of a syringe or a syringe needle that needs to be discarded.

In addition, the present invention may reduce manufacturing costs and simplify a manufacturing process.

### [Background Art]

In the case of a general disposable syringe, a user is frequently pricked with a needle and infected with pathogen while putting the needle in a needle storage cap after use and, when a needle should not be reused, a lot of cases in which disease is spread to unrelated patients due to reuse of needles are reported.

To prevent such unpredicted injury or disease infection of a syringe user, various forms of safe syringes have been proposed and, as representative examples of the safe syringe, a safe syringe disclosed in Korean Patent Publication Nos. 10-2003-0043209, 2001-0040365, and the like is proposed.

However, such a safe syringe is relatively expensive and a user is inconvenienced by using both hands when storing a needle.

In addition, the safe syringe disadvantageously has high separate manufacturing costs and has a complex manufacturing process.

### [Disclosure]

### [Technical Problem]

An object of the present invention devised to solve the problem lies in a syringe safe cap assembly for preventing a safe cap from falling to the ground to lastly stand itself according to a principle of a roly-poly toy and for enabling a syringe needle or a needle support to be easily inserted into the safe cap using only a hand that holds a syringe.

Another object of the present invention devised to solve the problem lies in a syringe safe cap assembly for enabling syringe reuse or basically preventing syringe reuse according to whether it is possible to reuse a syringe or pressure for inserting the syringe into a safe cap.

Another object of the present invention devised to solve the problem lies in a syringe safe cap assembly for simplifying a structure and manufacturing process thereof to reduce manufacturing costs, thereby enhancing economic efficiency.

### [Technical Solution]

The object of the present invention can be achieved by providing a syringe safe cap assembly including an accommodation cap into which at least a portion of a syringe needle or a syringe needle support is capable of being inserted and accommodated, and a support connected to the accommodation cap and configured to support the accommodation cap not to fall down with respect to a ground surface, wherein the supports includes a housing portion, at least a portion of an external surface of which has an outline of a curved line, and wherein, when the accommodation cap is put on a ground, the support guides the accommodation cap to immediately stand on a ground or, selectively, guides the accommodation cap to lastly stand on the ground while the accommodation cap rolls from side to side.

In another aspect of the present invention, provided herein is a syringe safe cap assembly including an accommodation cap into which at least a portion of a syringe needle or a syringe needle support is capable of being inserted and accommodated, and a support connected to the accommodation cap and configured to support the accommodation cap not to fall down with respect to a ground surface, wherein the support includes a mass center weight portion disposed at a lower portion of the support to provide a center of mass and connected to the accommodation cap, a lateral wall portion extending upward from a lateral surface of the mass center weight portion and configured in the form of a convex curved surface, and an opening portion disposed between the lateral wall portions, and wherein, when the accommodation cap is put on a ground, the support guides the accommodation cap to immediately stand on a ground or, selectively, guides the accommodation cap to lastly stand on the ground while the accommodation cap rolls from side to side.

In another aspect of the present invention, provided herein is a syringe safe cap assembly including an accommodation cap into which at least a portion of a syringe needle or a syringe needle support is capable of being inserted and accommodated, and a support connected to the accommodation cap and configured to support the accommodation cap not to fall down with respect to a ground surface, wherein the support includes, a center mass weight portion configured to provide a center of mass and connected to the accommodation cap, a lateral wall portion spaced apart from the mass center weight portion, connected to the accommodation cap to surround the mass center weight portion, and configured in the form of a convex curved surface, and an opening portion disposed at a lower portion and an internal portion of the lateral wall portion, and wherein, when the accommodation cap is put on a ground, the support guides the accommodation cap to immediately stand on a ground or, selectively, guides the accommodation cap to lastly stand on the ground while the accommodation cap rolls from side to side.

### [Advantageous Effects]

According to the present invention, a cap may be capable of covering a needle using only a hand that grips a syringe after a syringe user uses the syringe and, thus, the possibility that an opposite hand of the hand that grips the syringe is pricked with the needle may be originally prevented, thereby achieving user safe.

That is, a safe cap is always directed upward according to a principle of a roly-poly toy and, thus, a needle and a support may be easily inserted into the safe cap.

The principle of the roly-poly toy may be embodied by a curved surface portion of a support and a mass center weight portion in the support or by the mass center weight portion and a lateral wall portion connected thereto.

By virtue of a support to guide a safe cap assembly to always stand, even if a syringe user just puts the safe cap assembly on the ground without accurately maintaining a posture of the safe cap assembly to permit the safe cap assembly to stand, the safe cap assembly may automatically stand, thereby enhancing user convenience.

When a syringe needle needs to be reused, for example, when a syringe needs to be continuously used for a specific patient, a needle and a needle support may be slightly inserted and arranged into the safe cap and, then, may be re-taken and reused.

However, when a syringe may be pressed by predetermined pressure with respect to a safe cap assembly to originally prevent reuse of a syringe needle and to prevent another person from being infected with pathogen, a needle support may be moved downward to be caught by an engagement portion and, thus, the needle support may be prevented from being moved upward and it may be very difficult to re-take the needle and the needle support.

Accordingly, a reuse state or a discard state of a needle and a needle support may be easily embodied according to user selection.

An insert-guidance surface formed like a funnel-shaped tube may be provided at a mouth of an accommodation cap and, thus, a needle and a needle support may be easily inserted into the accommodation cap without accurately aiming at the mouth.

As such, when an accommodation cap is bent in a state in which upward movement is prevented, a portion of a needle is also bent and, thus, a discard state may be further clearly embodied and, accordingly, the discard state may be further clearly indicated.

According to the present invention, a support is open in forward and backward directions and, thus, a shape of a mold of the support may be simplified and the number of used molds may be reduced, thereby achieving economic efficiency.

### [Description of Drawings]

FIG. 1 is a perspective view of the present invention.
FIG. 2 is a side view of the present invention.
FIG. 3 is a perspective view of a lateral cross section of the present invention.
FIG. 4 is a side cross-sectional view of the present invention.
FIG. 5 is an enlarged perspective view of an insert-guidance surface according to the present invention.
FIGS. 6 and 7 are perspective views of a syringe safe cap assembly according to a second embodiment of the present invention.
FIG. 8 is a perspective view of a cross section of the syringe safe cap assembly according to the second embodiment of the present invention.
FIG. 9 is a plan view of the syringe safe cap assembly according to the second embodiment of the present invention.
FIG. 10 is a side view of the syringe safe cap assembly according to the second embodiment of the present invention.
FIG. 11 is a front view of the syringe safe cap assembly according to the second embodiment of the present invention.
FIG. 12 is a perspective view showing a state in which an accommodation cap is bent in the syringe safe cap assembly according to the second embodiment of the present invention.
FIG. 13 is a perspective view of a syringe safe cap assembly according to a third embodiment of the present invention.
FIG. 14 is a perspective view of a cross section of the syringe safe cap assembly according to the third embodiment of the present invention.
FIG. 15 is a plan view of the syringe safe cap assembly according to the third embodiment of the present invention.
FIG. 16 is a front view of the syringe safe cap assembly according to the third embodiment of the present invention.
FIG. 17 is a side view of the syringe safe cap assembly according to the third embodiment of the present invention.
FIG. 18 is a perspective view showing a state in which an accommodation cap is bent in the syringe safe cap assembly according to the third embodiment of the present invention.

### [Best Mode]

As those skilled in the art would realize, the described embodiments may be modified in various different ways and exemplary embodiments are described with reference to the accompanying drawings.

However, this is not intended to limit the present invention to particular modes of practice, and it is to be appreciated that all changes, equivalents, and substitutes that do not depart from the spirit and technical scope of the present invention are encompassed in the present invention.

The terms such as "first" and "second" are used herein merely to describe a variety of constituent elements, but the constituent elements are not limited by the terms.

The terms are used only for the purpose of distinguishing one constituent element from another constituent element.

For example, a first element may be termed a second element and a second element may be termed a first element without departing from the teachings of the present invention.

As used herein, the term "and/or" includes any and all combinations of one or more of the associated listed items

It will be understood that when an element, such as a layer, a region, or a substrate, is referred to as being "on", "connected to" or "coupled to" another element, it may be directly on, connected or coupled to the other element or intervening elements may be present.

In contrast, when an element is referred to as being "directly on," "directly connected to" or "directly coupled to" another element or layer, there are no intervening elements or layers present.

The terms used in the present specification are used for explaining a specific exemplary embodiment, not limiting the present invention.

The singular expressions in the present specification include the plural expressions unless clearly specified otherwise in context.

The terms such as "include" or "comprise" used herein may be construed to denote a certain characteristic, number, step, operation, constituent element, or a combination thereof, but may not be construed to exclude the existence of or a possibility of addition of one or more other characteristics, numbers, steps, operations, constituent elements, or combinations thereof

Hereinafter, the present invention will be described in detail by explaining exemplary embodiments of the invention with reference to the attached drawings. The same reference numerals in the drawings denote like elements, and a repeated explanation thereof will not be given.

Hereinafter, the present invention will be described with reference to the attached drawings.

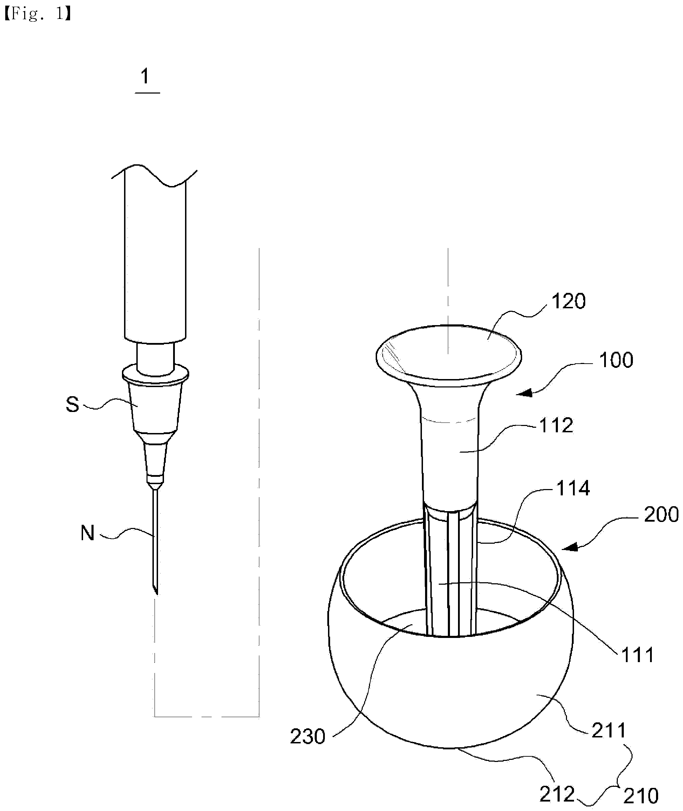

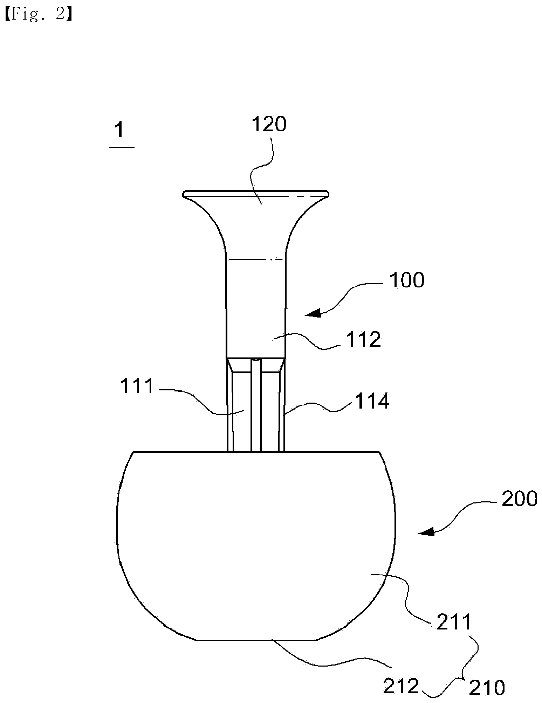

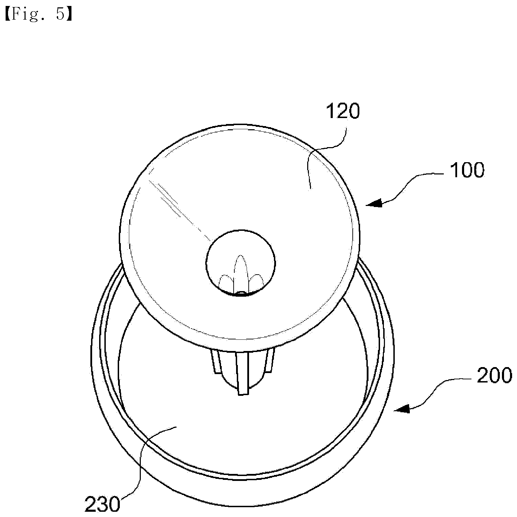

As shown in FIGS. 1 to 5, a syringe safe cap assembly 1 according to a first embodiment of the present invention may include an accommodation cap 100 into which a needle N of a syringe or a needle support S11 is inserted to be accommodated and a support portion 200 that is connected to a lower portion of the accommodation cap 100 and supports the accommodation cap 100 not to fall down to the ground surface.

The accommodation cap 100 may enable the needle N and the needle support S to be completely inserted and accommodated therein.

Accordingly, the accommodation cap 100 may be formed in a long shape in upward and downward directions and may include a needle accommodation space 111 and a needle support accommodation space 112 that are each formed therein.

The needle accommodation space 111 and the needle support accommodation space 112 may be connected to each other and the needle support accommodation space 111 may be formed at an upper portion and the needle accommodation space 112 may be formed at a lower portion.

A groove or protrusion for catching the needle support S may be formed to configure an engagement portion 113 on an internal wall for forming the needle support accommodation space 112.

When a protrusion is formed on an external surface of the needle support S, the protrusion may be inserted into the engagement portion 113 formed like a groove.

Alternatively, an external surface of the needle support may be caught and supported by the engagement 113 formed like a protrusion.

The engagement 113 may be integrally formed with the accommodation cap 100 on an internal surface of the accommodation cap 100 and may protrude or may be formed like a groove and may be caught and coupled to the needle support S by predetermined pressurization force to prevent the needle N and the needle support S from escaping from the accommodation cap.

Although described below, when a user holds a syringe with a needle installed therein with one hand and inserts the needle N and the needle support S into the accommodation cap 100, if the user slightly inserts the needle N and the needle support S into the accommodation cap 100, the needle support S may be put on the engagement portion 113 rather than being inserted into the engagement portion 113.

In this state, when the user re-holds and lifts a syringe body, the needle N and the needle support S may escape from the accommodation cap 100.

However, when the user holds a syringe and inserts the needle N and the needle support S into the accommodation cap 100, if the user inserts the needle N and the needle support S into the accommodation cap 100 by predetermined pressure or more, the needle support S may be caught by the engagement 113 and may fit tightly against the engagement portion 113.

In this state, even if the user re-holds and lifts the syringe body, the needle N and the needle support S may not escape from the accommodation cap 100.

That is, the needle N of a syringe may be capable of being reused (when an injection is applied to the same person) or may be prevented from being reused according to pressure that is applied by the user.

In addition, the accommodation cap 100 stands with respect to the ground surface by the support portion 200 and, thus, the needle N of a syringe may be inserted into the accommodation cap 100 with only one hand and it may not be necessary to hold the accommodation cap 100.

Accordingly, the problem in terms of the conventional art in which a hand holding a syringe accommodation cap is pricked with a syringe needle by mistake may be completely prevented because it is not necessary to hold the accommodation cap of the syringe needle with one hand, to hold a syringe body is held with another hand, and to insert the needle into the accommodation cap.

As described above, the accommodation cap 100 may include the needle support accommodation space 112 and the needle accommodation space 111 that are formed therein.

An external diameter of a lower portion that forms the needle accommodation space 111 may be smaller than that of an upper portion that forms the needle support accommodation space 112 and at least one rib 114 may be formed in upward and downward directions on an external portion of the lower portion that forms the needle accommodation space 111.

Here, the ribs 114 may be used for reinforcement.

That is, since the accommodation cap 100 is formed in a long shape compared with a diameter thereof, strength needs to be reinforced to sufficiently maintain the shape and, to this end, the ribs are arranged.

The plurality of ribs 114 may be provided, arranged along the lower portion of the accommodation cap 100, and spaced apart from each other.

An insert-guidance surface 120 may be formed at a mouth of the accommodation cap 100.

The insert-guidance surface 120 may be formed like a funnel-shaped tube and may have a diameter that is increased upward.

In this regard, when a user holds a syringe with one hand and puts the needle N and the needle support S in the mouth of the accommodation cap 100, the needle N and the needle support S may be guided according to guidance of the insert-guidance surface 120 to be lastly inserted into the mouth without accurately aiming at the mouth.

Accordingly, the insert-guidance surface 120 may have a lateral cross section that is inclined downward and may be formed toward the mouth.

The support portion 200 may be connected to the lower portion of the accommodation cap 100.

The support portion 200 may be provided in the form of a container or in a stereoscopic shape.

An external surface of the support portion 200 may include a curved surface portion 211 and a flat surface portion 212.

The flat surface portion 212 may be embodied on a central portion of an external surface of a bottom of the support portion 200 and the curved surface portion 211 that extends from the flat surface portion 212 in an outer direction may be formed around the flat surface portion 212.

The curved surface portion 211 may extend from the flat surface portion 212 in an outer direction and, in detail, may extend in an upward direction while maintaining a curved surface.

The flat surface portion 212 may permit the support portion 200 to definitely stand with respect to the ground surface.

When a user disturbs the center of mass of the accommodation cap 100 while inserting the needle N of a syringe or the needle support S into the accommodation cap 100, the curved surface portion 211 may prevent the syringe safe cap assembly 1 from laterally falling down while allowing the syringe safe cap assembly 1 to overall roll from side to side via an interaction with a mass center weight portion 230 to be described below and, lastly, may also permit the syringe safe cap assembly 1 to stand due to support of the flat surface portion 212.

The mass center weight portion 230 that provides a predetermined weight to function as the center of mass of the support portion 200 may be provided in the support portion 200.

A housing portion 210 including flat surface portion 212 and the curved surface portion 211 may form an external surface of the support portion 200 and the mass center weight portion 230 may be arranged in the housing portion 210.

Here, the housing portion 210 and the mass center weight portion 230 may be integrally formed or the mass center weight portion 230 may be separately inserted and arranged in a space in the housing portion 210.

A portion (a lower end portion) of the lower portion of the accommodation cap 100 may be connected to the mass center weight portion 230 or may be formed to be surrounded by the mass center weight portion 230.

Accurately, the lower end portion of the lower portion of the accommodation cap 100 may be arranged at the center of an upper surface of the mass center weight portion 230.

According to the above configuration, when a user puts the syringe safe cap assembly 1 on the ground, the flat surface portion 212 may directly contact the ground to stand and, even if the syringe safe cap assembly 1 is put to be slightly inclined, the flat surface portion 212 may stably contact the ground surface to be supported with respect to the ground due to the function of the curved surface portion 211 and the mass center weight portion 230 as an amplitude of a reciprocating motion of the syringe safe cap assembly 1 in right and left directions is lastly reduced while the syringe safe cap assembly 1 rolls from side to side.

However, when the housing portion 210 has a large size, in particular, when the curved surface portion 211 has a large area or size, even if the mass center weight portion 230 is not used, an amplitude of a reciprocating motion of the syringe safe cap assembly 1 in right and left directions is lastly reduced while the syringe safe cap assembly 1 rolls from side to side like a roly-poly toy and, thus, the flat surface portion 212 may stably and lastly contact the ground surface to be supported with respect to the ground surface.

That is, according to the present invention, the mass center weight portion 230 may be selectively arranged.

An operation of holding a syringe body and inserting the needle N and the needle support S into the accommodation cap 100 by a user in a state in which the syringe safe cap assembly 1 into which the needle N of a syringe or the needle support S is not inserted stands stably with respect to the ground surface is now described below.

When the user inserts the needle N or the needle support S into the accommodation cap 100 while accurately aiming at the mouth of the accommodation cap 100 and enables the syringe body to stand, the syringe safe cap assembly 1 may accommodate the needle N or the needle support S therein while maintaining a state in which the syringe safe cap assembly 1 stably stands without rolling from side to side undoubtedly.

However, when the user inserts the needle N or the needle support S into the accommodation cap 100 while obliquely putting the syringe body, an end portion of the needle N may first contact the insert-guidance surface 120 shaped like the funnel-shaped tube.

In this state, when the end portion of the needle N is moved downward according to guidance of the insert-guidance surface 120, the needle N may be inserted into the mouth of the accommodation cap 100 and, then, the needle support S may also be inserted into the mouth of the accommodation cap 100.

During such an insertion procedure, a state in which the accommodation cap 100 stands may be released and the accommodation cap 100 may roll from side to side.

However, curved surface 120 may guide a reciprocating motion of the syringe safe cap assembly 1 in right and left directions and the flat surface portion 212 may lastly and continuously contact the ground surface as an amplitude of the reciprocating motion is gradually reduced by the mass center weight portion 230, thereby enabling the syringe safe cap assembly 1 to stably stand via support of the flat surface portion 212.

As described above, when the user slightly put a syringe in the syringe safe cap assembly 1, the needle support S is slightly put on the engagement portion 113 and, thus, when the syringe body is re-lifted, the needle N of a syringe and the needle support S may escape from the accommodation cap 100 and the syringe may be reused.

Needless to say, during the procedure in which the syringe escapes from the accommodation cap 100, even if the syringe safe cap assembly 1 rolls from side to side, the syringe safe cap assembly 1 may return to a stand-up state soon after rolling from side to side according to a principle of a roly-poly toy.

When a user presses a syringe body with predetermined pressure to prevent a syringe needle from being reused, the needle support S may be held and caught by the engagement portion 113 or may be moved to a lower portion of the engagement portion 113.

In this case, when the needle support S is caught by the engagement portion 113, even if the user holds the syringe body and raises the syringe body upward, the needle N and the needle support S may be prevented from escaping from the accommodation cap 100.

Accordingly, in this case, reuse of the syringe may be originally prevented and, thus, a problem in terms of inappropriate reuse of a syringe may be completely prevented.

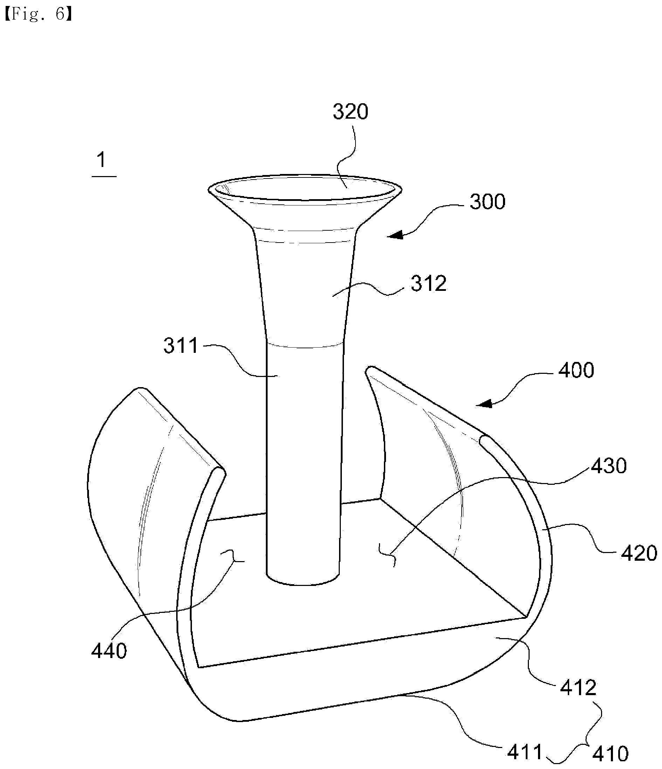

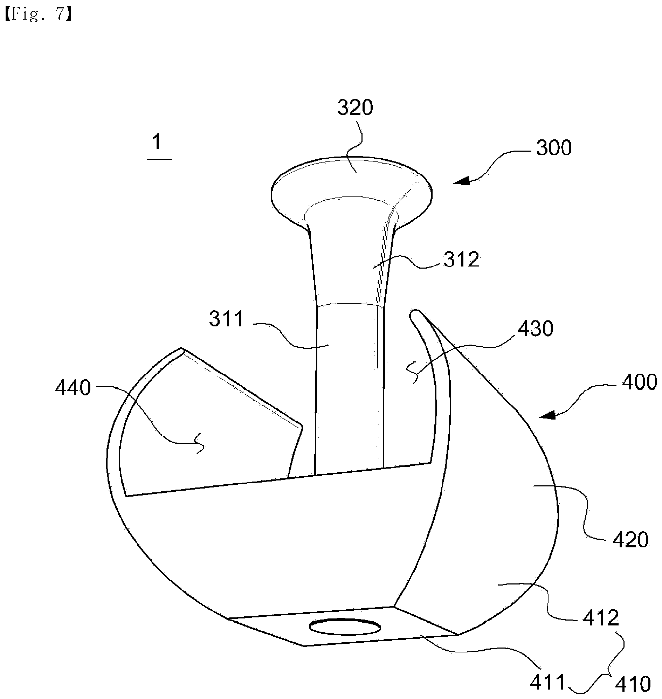

As shown in FIGS. 6 and 7, the syringe safe cap assembly 1 according to a second embodiment of the present invention may include an accommodation cap 300 into which a needle (not shown) of a syringe or a needle support (not shown) is inserted to be accommodated and a support 400 that is connected to a lower portion of the accommodation cap 300 and supports the accommodation cap 300 not to fall down to the ground surface.

The accommodation cap 300 may enable the needle and the needle support to be completely inserted and accommodated therein.

Accordingly, the accommodation cap 300 may be formed in a long shape in upward and downward directions and may include a needle accommodation space 311 and a needle support accommodation space 312 that are each formed therein.

The needle accommodation space 311 and the needle support accommodation space 312 may be connected to each other and the needle support accommodation space 311 may be formed at an upper portion and the needle accommodation space 312 may be formed at a lower portion.

A groove or protrusion for catching the needle support may be formed to configure an engagement portion (not shown) on an internal wall for forming the needle support accommodation space 312.

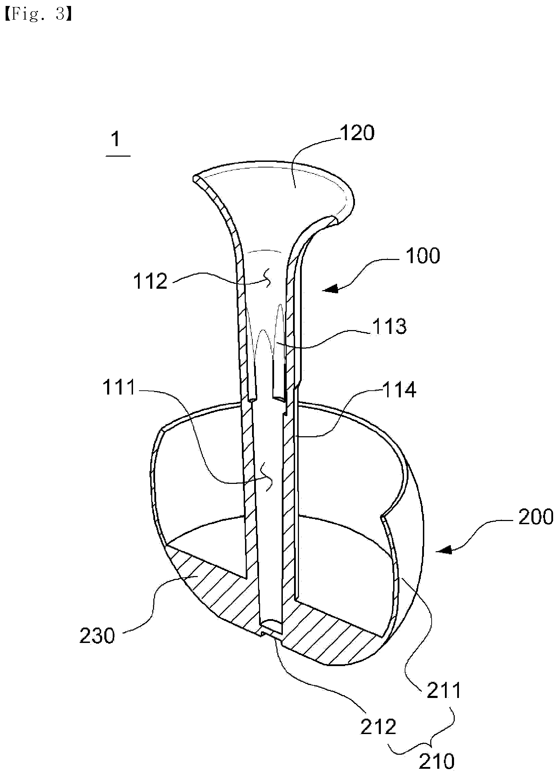

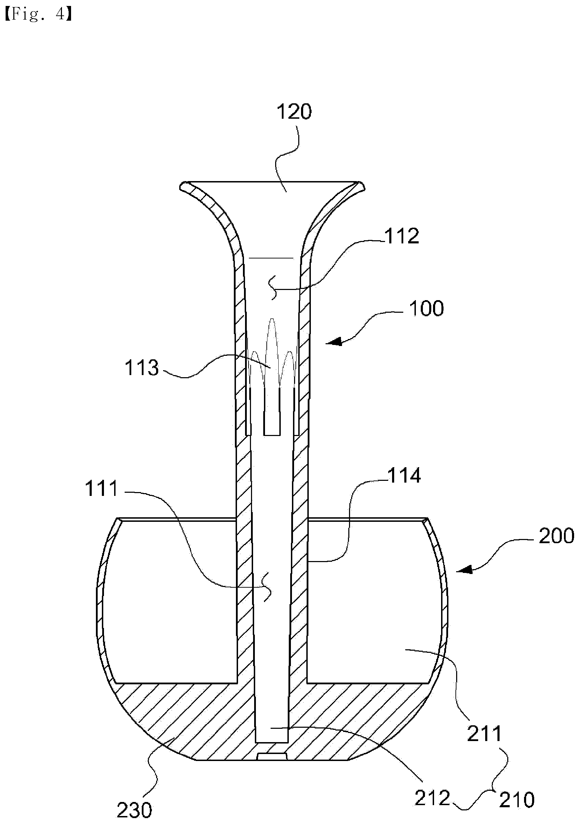

The engagement portion according to the second embodiment and engagement portion according to third and fourth embodiments to be described below may have the same shape and function of the engagement portion 113 (refer to FIGS. 3 and 4) according to the first embodiment.

When a protrusion is formed on an external surface of the needle support, the protrusion may be inserted into the engagement portion formed like a groove. Alternatively, an external surface of the needle support may be caught and supported by the engagement portion formed like a protrusion.

Although described below, when a user holds a syringe with a needle installed therein with one hand and inserts the needle and the needle support into the accommodation cap 300, if the user slightly inserts the needle and the needle support into the accommodation cap 300, the needle support may be put above the engagement portion rather than being inserted into the engagement portion.

In this state, when the user re-holds and lifts a syringe body, the needle and the needle support may escape from the accommodation cap 300.

However, when the user holds a syringe and inserts the needle and the needle support into the accommodation cap 300, if the user inserts the needle and the needle support into the accommodation cap 300 by predetermined pressure or more, the needle support may be caught by the engagement portion and may fit tightly against the engagement portion.

In this state, even if the user re-holds and lifts the syringe body, the needle and the needle support may not escape from the accommodation cap 300.

That is, the syringe needle may be capable of being reused (when an injection is applied to the same person) or may be prevented from being reused according to pressure that is applied by the user.

In addition, the accommodation cap 300 stands with respect to the ground surface by the support 400 and, thus, the syringe needle may be inserted into the accommodation cap 300 with only one hand and it may not be necessary to hold the accommodation cap 300.

Accordingly, the problem in terms of the conventional art in which a hand holding a syringe accommodation cap is pricked with a syringe needle by mistake may be originally prevented because it is not necessary to hold the accommodation cap of the syringe needle with one hand, to hold a syringe body is held with another hand, and to insert the needle into the accommodation cap.

As described above, the accommodation cap 300 may include the needle support accommodation space 312 and the needle accommodation space 311.

An external diameter of a lower portion that forms the needle accommodation space 311 may be smaller than that of an upper portion that forms the needle support accommodation space 312.

An insert-guidance surface 320 may be formed at a mouth of the accommodation cap 300.

The insert-guidance surface 320 may be formed like a funnel-shaped tube and may have a diameter that is increased upward.

In this regard, when a user holds a syringe with one hand and puts the needle and the needle support in the mouth of the accommodation cap 300, the needle and the needle support may be guided according to guidance of the insert-guidance surface 320 to be lastly inserted into the mouth without accurately aiming at the mouth.

Accordingly, the insert-guidance surface 320 may have a lateral cross section that is inclined downward and may be formed toward the mouth.

The support 400 may be connected to the lower portion of the accommodation cap 300.

The support 400 may employ a principle of a roly-poly toy and, thus, even if the accommodation cap 300 and the support 400 is unstably put (i.e., the accommodation cap 300 and the support 400 is put obliquely but not vertically), the accommodation cap 300 and the support 400 may lastly and stably stand in a perpendicular direction to the ground surface without falling down while rolling from side to side.

The support 400 may include a mass center providing portion 410 and a lateral wall portion 420 that extends in an upward direction from a lateral surface of the mass center providing portion 411.

The lateral wall portion 420 may extend while forming a convex wall in an upward direction from opposite lateral surfaces of the mass center providing portion 410, may be formed to face each other, and may be spaced apart from each other.

In addition, opening portions 430 may be provided at front and rear sides of the support 400 and a space portion 440 may be formed between the front opening portion and the rear opening portion.

As such, since the support 400 is open in two directions, an internal structure of a manufacturing mold thereof and the number of manufacturing molds may be simplified, thereby enhancing economic efficiency.

The mass center providing portion 410 may be provided in the form of a filled mass to provide the stable center of mass and may rapidly and vertically stand even if the safe cap assembly rolls from side to side.

The mass center providing portion 410 may include a curved surface portion 411 that is formed to be convex at a lateral surface thereof and a flat surface portion 412 that is formed to be flat on a bottom.

The curved surface portion 411 may extend from the flat surface portion 412 in an outer direction and, in detail, may extend in an upward direction while maintaining an outline of a curved surface.

The flat surface portion 412 may permit the support 400 to definitely stand with respect to the ground surface.

When a user disturbs the center of mass of the accommodation cap 300 while inserting a syringe needle or a needle support into the accommodation cap 300, the curved surface portion 411 may prevent the syringe safe cap assembly 1 from laterally falling down while allowing the syringe safe cap assembly 1 to overall roll from side to side and, lastly, may also permit the syringe safe cap assembly 1 to stand due to support of the flat surface portion 412.

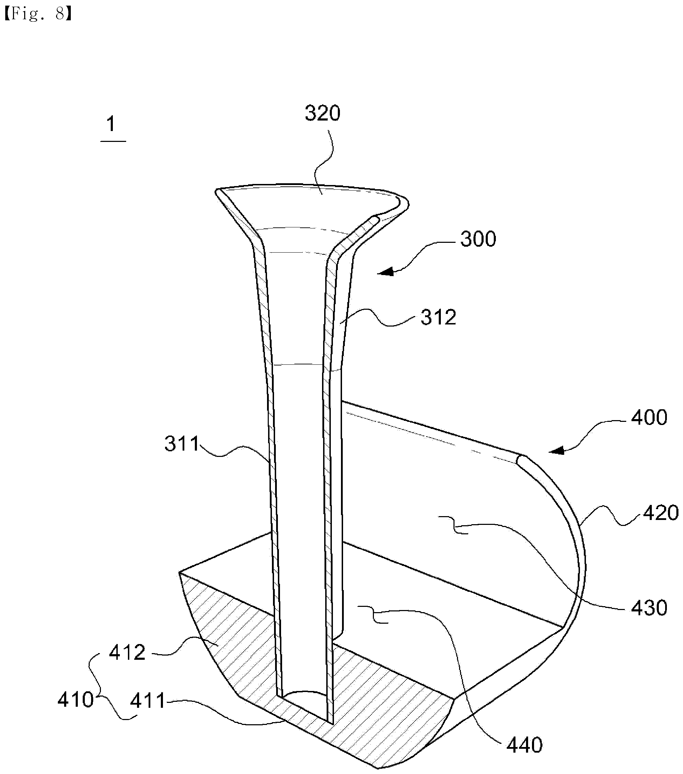

As shown in FIG. 8, a lower end portion of the accommodation cap 300 may be connected to the mass center providing portion 410 and, in detail, the accommodation cap 300 and the mass center providing portion 410 may be integrally formed as one injected material.

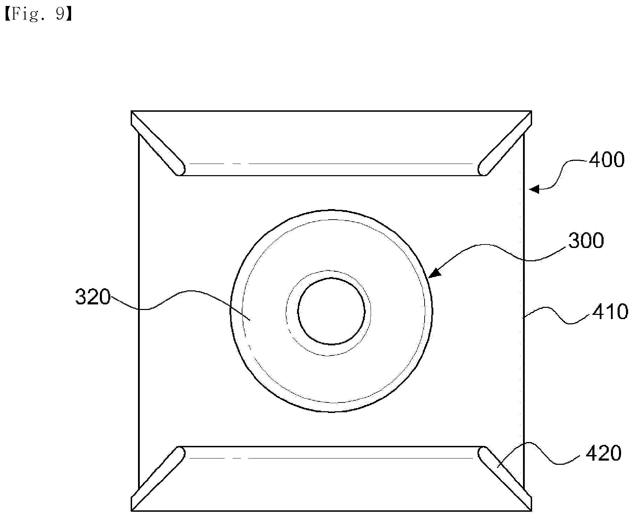

As shown in FIG. 9, the accommodation cap 300 may be arranged at the center of the support and may not be inclined to one side. This is because the syringe safe cap assembly 1 may be prevented from falling down even if the syringe safe cap assembly 1 rolls from side to side according to a principle of a roly-poly toy.

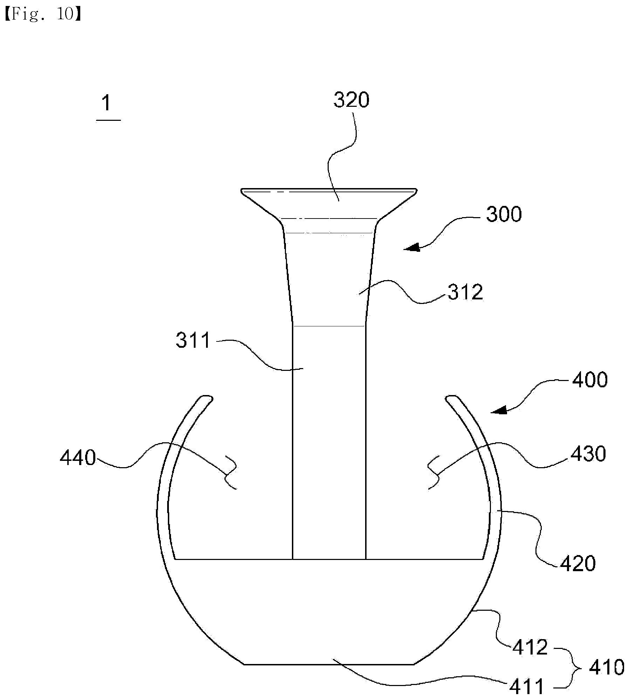

As shown in FIG. 10, the lateral wall portion 420 may further extend from the curved surface portion 412 in an upward direction and have a portion that is convex outward and an upper portion of the convex portion is directly inward.

Accordingly, an internal between the opposite lateral wall portions 420 may be maximum at the convex portion and may be reduced upward, and upper end portions of opposite lateral wall portions 420 may be spaced apart from each other.

When the opening portions 430 are formed at front and rear sides of the support 400 and the space portion 440 may be formed between opening portions 430, the front and rear side of the support 400 may be connected therethrough. Accordingly, a mold structure may be simplified.

Accordingly, when the support 400 and the accommodation cap 300 are prepared in the integral form via injection, production costs may be lowered due to lowered manufacturing costs of a mold.

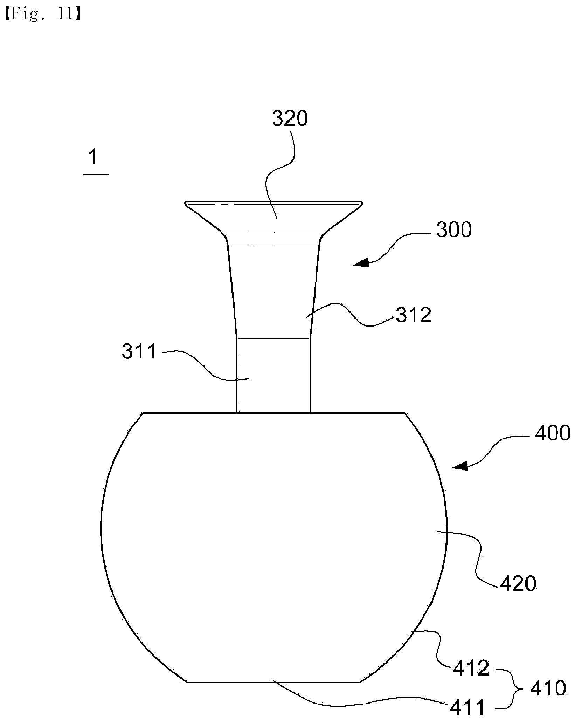

As shown in FIG. 11, with regard to curved shapes that form opposite lateral surfaces of the lateral wall portion 420 and the curved surface portion 412 , the lateral wall portion 420 and the curved surface portion 411 may be formed to be convex in opposite outer directions, may have the convex portion, a width of which is maximum in right and left directions, and may be tapered upward and downward from the convex portion.

Accordingly, even if the lateral wall portion 420 or the curved surface portion 412 contacts the ground while the syringe safe cap assembly 1 rolls from side to side, the syringe safe cap assembly 1 may lastly and stably stand in a perpendicular direction to the ground surface without falling down while rolling from side to side due to the curved shape as an amplitude of a reciprocating motion of the syringe safe cap assembly 1 in right and left directions is lastly reduced while the syringe safe cap assembly 1 rolls from side to side.

An operation of holding a syringe body and inserting the needle and the needle support into the accommodation cap 300 by a user in a state in which the syringe safe cap assembly 1 into which the syringe needle or the syringe support is not inserted stands stably with respect to the ground surface is now described below.

When the user inserts the needle or the needle support into the accommodation cap 300 while accurately aiming at the mouth of the accommodation cap 300 and enables the syringe body to stand, the syringe safe cap assembly 1 may accommodate the needle or the needle support therein while maintaining a state in which the syringe safe cap assembly 1 stably stands without rolling from side to side undoubtedly.

However, when the user inserts the needle or the needle support into the accommodation cap 300 while obliquely putting the syringe body, an end portion of the needle may first contact the insert-guidance surface 320 shaped like a funnel-shaped tube.

In this state, when the end portion of the needle is moved downward according to guidance of the insert-guidance surface 320, the needle may be inserted into the mouth of the accommodation cap 300 and, then, the needle support may also be inserted into the mouth of the accommodation cap 300.

During such an insertion procedure, a state in which the accommodation cap 300 stands may be released and the accommodation cap 300 may roll from side to side.

However, the lateral wall portion 420 and the curved surface portion 412 may guide a reciprocating motion of the syringe safe cap assembly 1 in right and left directions and flat surface portion 411 may lastly and continuously contact the ground surface as an amplitude of the reciprocating motion is gradually reduced by the mass center providing portion 410 , thereby enabling the syringe safe cap assembly 1 to stably stand via support of the flat surface portion 411 .

As described above, when the user slightly put a syringe into the syringe safe cap assembly 1, the needle support is slightly put on the engagement portion and, thus, when the syringe body is re-lifted, the syringe needle and the needle support may escape from the accommodation cap 300 and the syringe may be reused.

Needless to say, during the procedure in which the syringe escapes from the accommodation cap 300, even if the syringe safe cap assembly 1 rolls from side to side, the syringe safe cap assembly 1 may return to a stand-up state soon after rolling from side to side according to a principle of a roly-poly toy.

When a user presses a syringe body with predetermined pressure to prevent a syringe needle from being reused, the needle support may be held and caught by the engagement portion or may be moved to a lower portion of the engagement portion.

In this case, when the needle support is caught by the engagement portion, even if the user holds the syringe body and raises the syringe body upward, the needle and the needle support may be prevented from escaping from the accommodation cap 300.

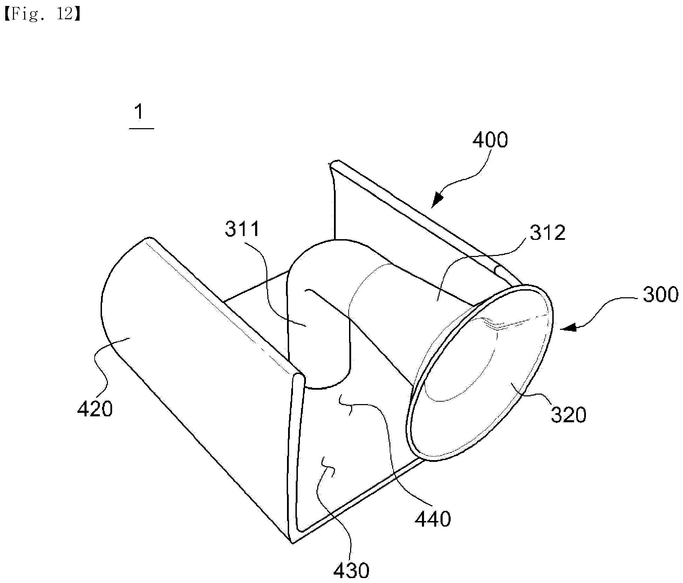

In this state, when the accommodation cap 300 is bent to form an accommodation cap formed like 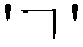 as shown in FIG. 12 and, accordingly, a needle inserted into the accommodation cap shown in FIG. 12 is also bent.

To easily bend the accommodation cap 300, a wall thickness of the bent portion of the accommodation cap 300 may be thinner than other portions.

In this state, reuse of the syringe may be originally prevented and, thus, a problem in terms of inappropriate reuse of a syringe may be originally prevented.

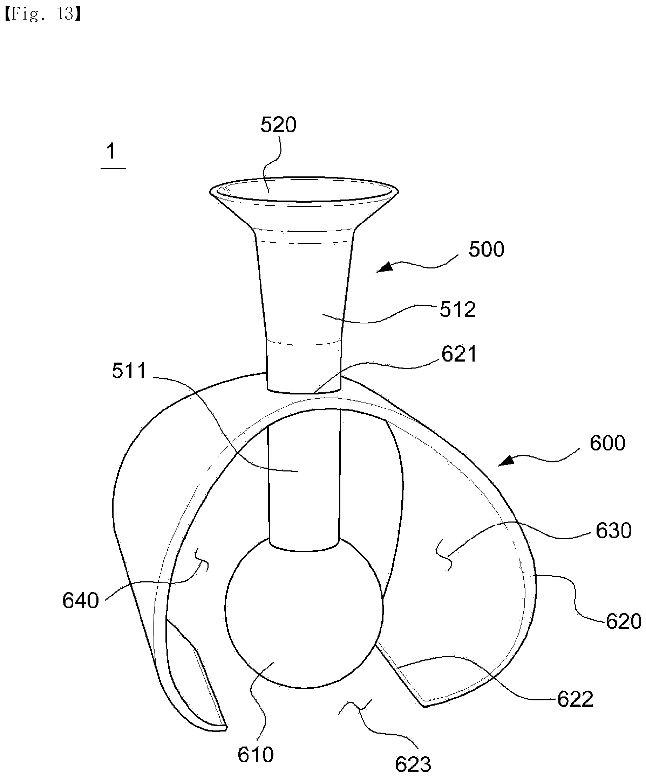

FIGS. 13 to 18 are diagrams for explanation of a third embodiment of the present invention.

As shown in FIG. 13, the syringe safe cap assembly 1 according to the third embodiment of the present invention may include an accommodation cap 500 into which a needle (not shown) of a syringe or a needle support (not shown) is inserted to be accommodated and a support 600 that is connected to the accommodation cap 500 and supports the accommodation cap 500 not to fall down to the ground surface.

The accommodation cap 300 may be formed in a long shape in upward and downward directions and may include a needle accommodation space 511 and a needle support accommodation space 512 that are each formed therein.

The needle accommodation space 511 and the needle support accommodation space 512 may be connected to each other and the needle support accommodation space 511 may be formed at an upper portion and the needle accommodation space 512 may be formed at a lower portion.

Among components of the accommodation cap 500 according to the present embodiment, a detailed description of functions of the same components (e.g., the needle accommodation space 511, the needle support accommodation space 512, and an insert-guidance surface 520) as that of the accommodation cap 300 according to the second embodiment is omitted to avoid repetition.

The support 600 may be largely classified into two portions.

The support 600 may include a mass center providing portion 610 formed like a sphere at a lower end portion of the accommodation cap 500 and a lateral wall portion 620 that is spaced apart from the mass center providing portion 610 to surround the mass center providing portion 610.

The mass center providing portion 610 may be integrally formed with the lower end portion of the accommodation cap 500 and may be provided in the form of a filled mass.

Due to a weight of the mass center providing portion 610, the accommodation cap 500 may easily stand.

The lateral wall portion 620 may surround a lateral surface and upper portion of the mass center providing portion 610 and may have an open lower portion.

That is, lower end portions 622 of the lateral wall portions 620 may be spaced apart from each other and the mass center providing portion 610 may be arranged in the spaced space.

The lower end portion 622 of the lateral wall portion 620 and a lower surface of the mass center providing portion 610 may be arranged on the same plane to support the syringe safe cap assembly 1.

In this case, multipoint support may be possible by the lower end portion 622 of the lateral wall portion 620, the lower surface of the mass center providing portion 610, and the like, thereby stably supporting the syringe safe cap assembly 1.

That is, the lower end portion 622 and the lower surface of the mass center providing portion 610 may function as the flat surface portion 411 according to the first embodiment.

The lateral wall portions 620 may extend to form a convex wall at opposite lateral surfaces of the mass center providing portion 610 and may have upper portions that are connected to each other and lower portions that are spaced apart from each other.

A connection portion 621 connected to the accommodation cap 500 may be provided at a lower portion of the lateral wall portion 620.

The lateral wall portion 620 may be integrally manufactured with the accommodation cap 500 via a single mold.

Opening portions 630 may be formed at front and rear sides of the support 600 and a space portion 640 may be formed between the front and rear opening portions.

A spaced space portion 623 may be formed below the support 600 and, as described above, may form the spaced space between the lower end portions 622 of the lateral wall portions 620.

According to the third embodiment of the present invention, since the support 600 is open in forward, backward, downward directions, an internal structure of a manufacturing mold thereof and the number of manufacturing molds may be simplified, thereby enhancing economic efficiency.

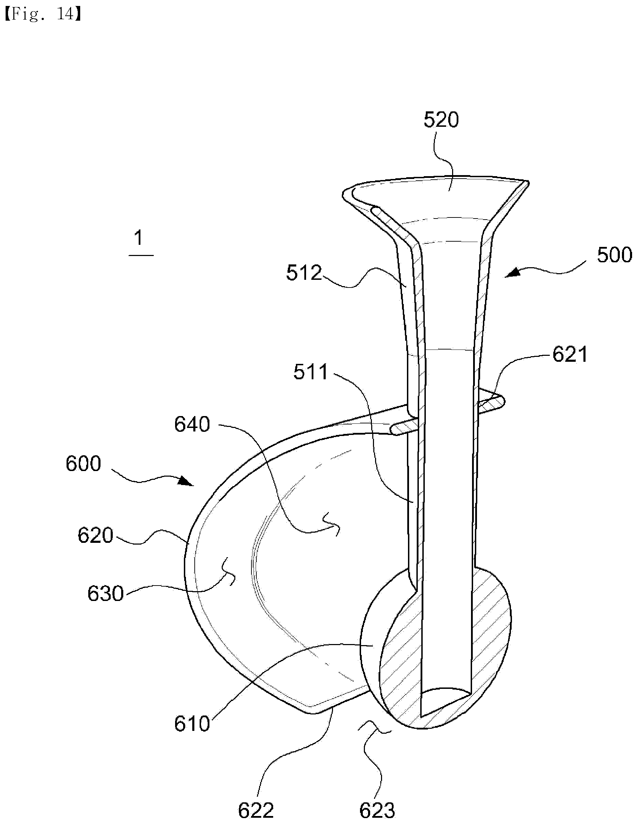

As shown in FIG. 14, the mass center providing portion 610 may be provided in the form of a mass formed like a filled sphere to provide the stable center of mass and, even if the safe cap assembly rolls from side to side, the safe cap assembly may rapidly and vertically stand.

The mass center providing portion 610 may be connected to the accommodation cap 500 and a lower region of the needle accommodation space 511 provided in the accommodation cap 500 may also extend to an internal portion of the mass center providing portion 610.

The lower end portion of the accommodation cap 500 may be connected to the mass center providing portion 610 and, in detail, the accommodation cap 500 and the mass center providing portion 610 may be integrally formed as one injected material.

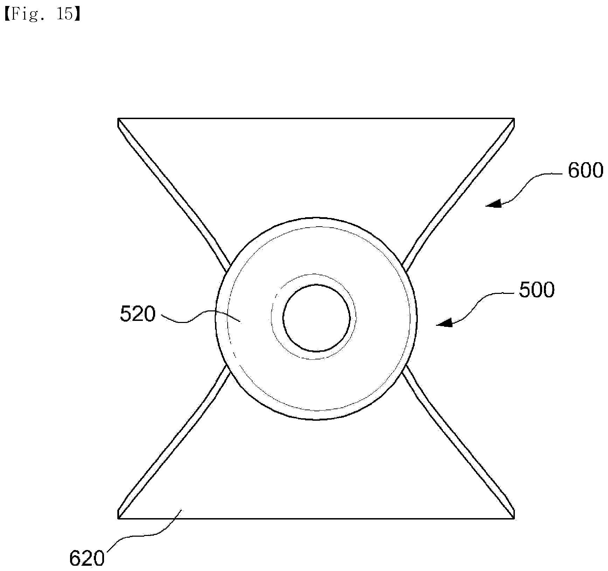

As shown in FIG. 15, the accommodation cap 500 may be arranged at the center of the support and may not be inclined to one side. This is because the syringe safe cap assembly 1 may be prevented from falling down even if the syringe safe cap assembly 1 rolls from side to side according to a principle of a roly-poly toy.

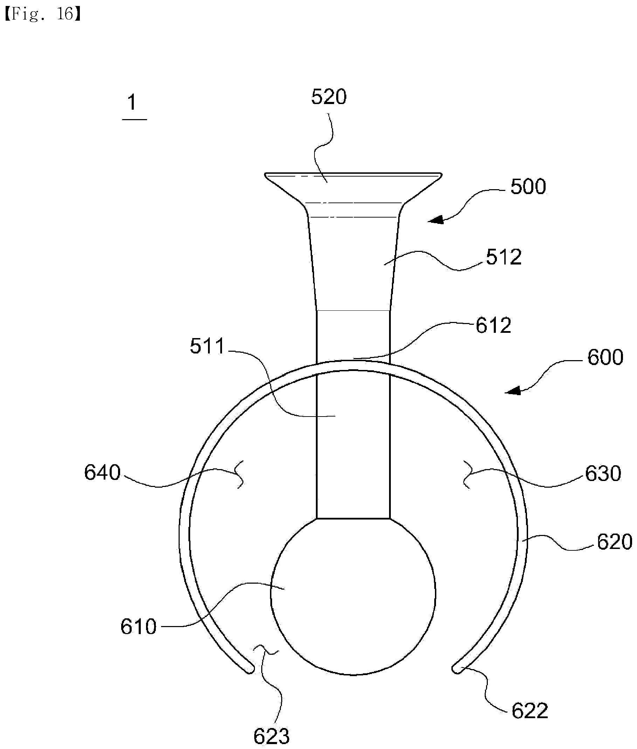

As shown in FIG. 16, the lateral wall portion 620 may be surrounded in the form of a curved line of a circular arc while being spaced apart from the mass center providing portion 610.

An internal between the opposite lateral wall portions 620 may be maximum at the convex portion, may be reduced upward, upper end portions of the lateral wall portions 620 may be connected, and lower end portions of the lateral wall portions 620 may be spaced part from each other.

Accordingly, the syringe safe cap assembly 1 may be supported by the two lower end portions 622 of the lateral wall portions and a lowermost surface of the mass center providing portion 610 and may rapidly stand even if the syringe safe cap assembly 1 rolls from side to side.

The opening portions 630 may be formed at front and rear sides of the support 600 and the space portion 640 and may be formed between the front and rear opening portions and, thus, the front and rear side of the support 600 may be connected therethrough. Accordingly, a mold structure may be simplified.

Accordingly, when the support 600 and the accommodation cap 500 are prepared in the integral form via injection, production costs may be lowered due to lowered manufacturing costs of a mold.

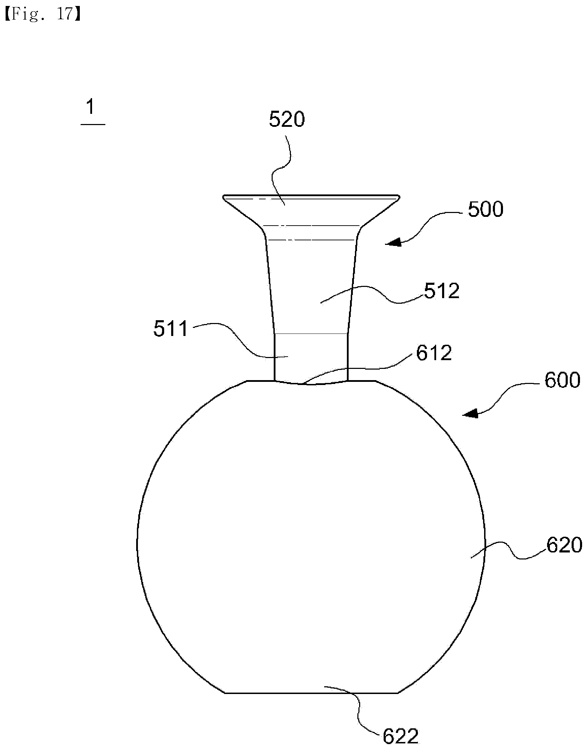

As shown in FIG. 17, with regard to curved shapes that form opposite lateral surfaces of the lateral wall portion 620, the lateral wall portion 620 may be formed to be convex in opposite outer directions, may have the convex portion, a width of which is maximum in right and left directions, and may be tapered upward and downward from the convex portion.

Accordingly, even if the lateral wall portion 620 contacts the ground while the syringe safe cap assembly 1 rolls from side to side, the syringe safe cap assembly 1 may lastly and stably stand to the ground surface without falling down while rolling from side to side due to the curved shape as an amplitude of a reciprocating motion of the syringe safe cap assembly 1 in right and left directions is lastly reduced while the syringe safe cap assembly 1 rolls from side to side.

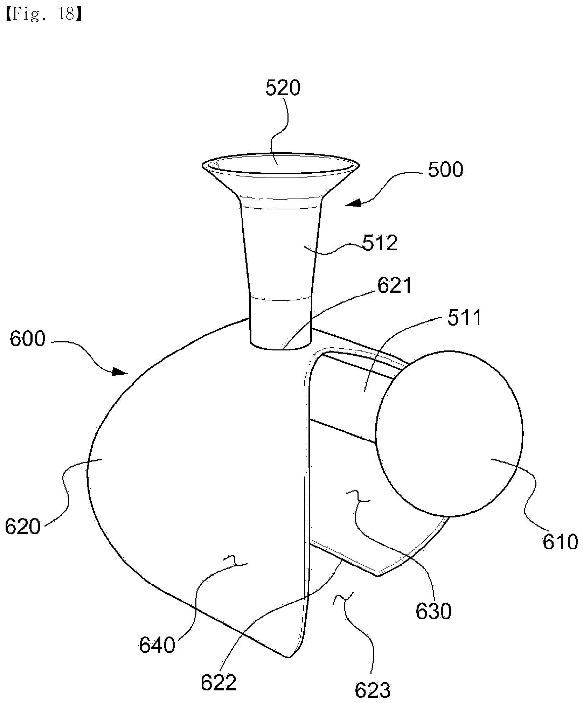

As shown in FIG. 18, when the accommodation cap 500 is bent while a needle is inserted into the accommodation cap 500, a potion of the accommodation cap 500, which is surrounded by the lateral wall portion 620 and connected to the mass center providing portion 610, may be bent and directed in a lateral direction and, accordingly, the needle may also be bent to completely prevent reuse of the needle.

The method of accommodating a syringe for reuse of a needle or the method of accommodating a syringe for prevention of reuse of a needle in the third embodiment is the same as in the first and second embodiments and, thus, a detailed description thereof is omitted to avoid repetition.

While the present invention has been particularly shown and described with reference to exemplary embodiments thereof, it will be understood by those of ordinary skill in the art that various changes in form and details may be made therein without departing from the spirit and scope of the present invention as defined by the following claims.

Thus, it is intended that the present invention cover the modifications and variations of this invention provided they come within the scope of the appended claims and their equivalents.

## Claims

1. A syringe safe cap assembly comprising:
an accommodation cap into which at least a portion of a syringe needle or a syringe needle support is capable of being inserted and accommodated; and
a support connected to the accommodation cap and configured to support the accommodation cap not to fall down with respect to a ground surface,
wherein the supports comprises a housing portion, at least a portion of an external surface of which has an outline of a curved line; and
wherein, when the accommodation cap is put on a ground, the support guides the accommodation cap to immediately stand on a ground or, selectively, guides the accommodation cap to lastly stand on the ground while the accommodation cap rolls from side to side.

2. The syringe safe cap assembly of claim 1, further comprising a mass center weight portion disposed in the housing portion and configured to provide a center of mass of the support to prevent the accommodation cap and the support from falling down.

3. The syringe safe cap assembly of claim 1, wherein the housing portion comprises:
a flat surface portion formed on a central portion of a bottom of the housing portion; and
a curved surface portion extending in an outer direction of the flat surface portion to have a predetermined curvature.

4. The syringe safe cap assembly of claim 2, wherein the housing portion comprises a predetermined space portion formed therein; and
wherein the mass center weight portion is accommodated in the space portion.

5. The syringe safe cap assembly of any one of claims 1 to 3, wherein a lower portion of the accommodation cap is configured with a predetermined length to accommodate a needle; and
wherein a portion of the lower portion of the accommodation cap is connected to the mass center weight portion or is surrounded by the mass center weight portion.

6. The syringe safe cap assembly of any one of claims 1 to 3, further comprising an insert-guidance surface arranged at a mouth of the accommodation cap and configured to have an external diameter that increases upward to allow the syringe needle or the syringe needle support to be easily inserted into the mouth of accommodation cap.

7. The syringe safe cap assembly of claim 1, further comprising an engagement portion that is integrally formed with the accommodation cap on an internal surface of the accommodation cap, protrudes or is formed like a groove, is caught and coupled to the needle support by predetermined pressurization force to prevent the needle and the needle support from escaping from the accommodation cap.

8. A syringe safe cap assembly comprising:
an accommodation cap into which at least a portion of a syringe needle or a syringe needle support is capable of being inserted and accommodated; and
a support connected to the accommodation cap and configured to support the accommodation cap not to fall down with respect to a ground surface,
wherein the support comprises,
a mass center weight portion disposed at a lower portion of the support to provide a center of mass and connected to the accommodation cap,
a lateral wall portion extending upward from a lateral surface of the mass center weight portion and configured in the form of a convex curved surface, and
an opening portion disposed between the lateral wall portions; and
wherein, when the accommodation cap is put on a ground, the support guides the accommodation cap to immediately stand on a ground or, selectively, guides the accommodation cap to lastly stand on the ground while the accommodation cap rolls from side to side.

9. The syringe safe cap assembly of claim 8, wherein the mass center weight portion comprises, a flat surface portion formed to be flat on a bottom surface, and a curved surface portion extending in an outer direction of the flat surface portion to have a predetermined curvature; and
wherein the curved surface portion is connected to the lateral wall portion.

10. The syringe safe cap assembly of claim 8, wherein the opening portion is formed at front and rear sides of the support and a space portion is formed between the front opening and the rear opening.

11. The syringe safe cap assembly of claim 9, wherein a lateral edge of the lateral wall portion and a lateral edge of the curved surface portion are connected to each other and an outline of a connected portion is convex at a central portion thereof.

12. A syringe safe cap assembly comprising:
an accommodation cap into which at least a portion of a syringe needle or a syringe needle support is capable of being inserted and accommodated; and
a support connected to the accommodation cap and configured to support the accommodation cap not to fall down with respect to a ground surface,
wherein the support comprises, a center mass weight portion configured to provide a center of mass and connected to the accommodation cap, a lateral wall portion spaced apart from the mass center weight portion, connected to the accommodation cap to surround the mass center weight portion, and configured in the form of a convex curved surface, and an opening portion disposed at a lower portion and an internal portion of the lateral wall portion; and
wherein, when the accommodation cap is put on a ground, the support guides the accommodation cap to immediately stand on a ground or, selectively, guides the accommodation cap to lastly stand on the ground while the accommodation cap rolls from side to side.

13. The syringe safe cap assembly of claim 12, wherein supporting force with respect to the same plane is provided by an edge of a lower surface of the lateral wall portion and a lower portion of the mass center weight portion to permit the accommodation cap to stand.

14. The syringe safe cap assembly of claim 12, wherein an outline of an edge of a lateral surface of the lateral wall portion is convex at a central portion of the lateral wall portion.

15. The syringe safe cap assembly of any one of claims 8 and 12, further comprising an engagement portion that is integrally formed with the accommodation cap on an internal surface of the accommodation cap, protrudes or is formed like a groove, is caught and coupled to the needle support by predetermined pressurization force to prevent the needle and the needle support from escaping from the accommodation cap.
